(19) Europäisches Patentamt
European Patent Office
Office européen des brevets

(11) Veröffentlichungsnummer: **0 249 164 B1**

(12) **EUROPÄISCHE PATENTSCHRIFT**

(45) Veröffentlichungstag der Patentschrift: **30.10.91**

(51) Int. Cl.[5]: **C07C 215/08**, C11D 1/62

(21) Anmeldenummer: **87108162.6**

(22) Anmeldetag: **05.06.87**

# (54) Neue Kationtenside auf der Basis von quartären Ammoniumverbindungen und ihre Verwendung in Reinigungsmitteln.

(30) Priorität: **13.06.86 DE 3620011**

(43) Veröffentlichungstag der Anmeldung:
**16.12.87 Patentblatt 87/51**

(45) Bekanntmachung des Hinweises auf die Patenterteilung:
**30.10.91 Patentblatt 91/44**

(84) Benannte Vertragsstaaten:
**AT BE CH DE ES FR GB IT LI NL SE**

(56) Entgegenhaltungen:
**EP-A- 0 012 215**
**EP-A- 0 127 131**
**FR-A- 2 319 421**
**US-A- 3 636 114**
**EP-A- 0 075 065**
**EP-A- 0 189 085**
**US-A- 3 503 890**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Geke, Jürgen, Dr.**
**Stoffeler Damm 108**
**W-4000 Düsseldorf 1(DE)**
Erfinder: **Rutzen, Horst, Dr.**
**Falkenweg 12**
**W-4018 Langenfeld(DE)**

**EP 0 249 164 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue, verbesserte Kationtenside auf der Basis von quartären Ammoniumverbindungen und die Verwendung derartiger Kationtenside in industriell angewendeten Reinigerlösungen.

Für die Reinigung von Halbfertigteilen oder Fertigteilen in der industriellen Produktion, beispielsweise von Kraftfahrzeugteilen aus Eisen oder Stahl, werden wäßrige Lösungen verwendet, die Tenside neben weiteren Hilfsstoffen, wie Buildersubstanzen, Komplexbildnern, organischen oder anorganischen Korrosionsschutzmitteln und gegebenenfalls weiteren Substanzen enthalten. So werden in der DE-A-27 12 900 und der DE-A-32 47 431 Verfahren vorgeschlagen, in denen im alkalischen pH-Wert-Bereich neben weiteren Reinigerbestandteilen quartäre Ammoniumverbindungen als Kationtenside verwendet werden, in denen an das Ammonium-Stickstoffatom organische Reste, insbesondere Alkylreste unterschiedlicher Kettenlänge, gebunden sind. Gegenionen der verwendeten Ammonium-Kationen sind beispielsweise Anionen wie Chlorid, Sulfat oder Methylsulfat, die als Korrosion verursachende Anionen bekannt sind. Der notwendige Gehalt an derartigen Anionen fördert unerwünschterweise an Anlagenteilen und behandelten Metalloberflächen die Korrosion. Dies ist insbesondere bei der Behandlung von Metalloberflächen mit wäßrigen Produkten von außerordentlichem Nachteil, insbesondere dann, wenn höhere Anwendungskonzentrationen an Kationtensiden gewünscht sind. Dabei tritt Korrosion nicht nur langfristig bei der Zwischenlagerung behandelter Teile ein, sondern auch unmittelbar bei der Behandlung entsprechender Flächen mit den wäßrigen Anwendungslösungen.

Auch in der DE-A-30 48 642 werden Tensidgemische zur Reinigung von Flaschen und sonstigen Gegenständen mit harten Oberflächen (Porzellan, Kunststoff, Metall) offenbart, die kationische Tenside auf der Basis von Ammoniumverbindungen enthalten. Ein Nachteil dieser Tenside ist jedoch auch, daß sie als Anionen Chlorid, Bromid oder Methylsulfat enthalten. Auch in diesem Fall wirken die Anionen einem als Folge der Behandlung mit derartigen Tensidgemischen erwünschten Korrosionsschutz auf Anlagenteilen (z.B. Spülmaschinen) und behandelten Oberflächen entgegen.

Verfahren zur Herstellung von quartären Ammoniumverbindungen, die wenigstens einen langkettigen Hydroxyalkylrest enthalten, durch Umsetzung des Salzes eines tertiären Amins und einer organischen Säure in Wasser mit einer einen Hydroxyalkylrest einführenden endständigen Epoxidverbindung bei Normaldruck, Temperaturen zwischen 40 und 100 °C und einem pH-Wert von wenigstens 7 sind aus der DE-A-33 21 608 bekannt. Die resultierenden quartären Ammoniumverbindungen konnten jedoch ebenfalls die hohen Anforderungen hinsichtlich Anwendungsfreundlichkeit und Korrosionsschutz nicht erfüllen, die an handelsübliche kationische Tenside gestellt werden. So sind die Anionen zahlreicher organischer Säuren zur Kationtensid-Gegenionbildung nicht geeignet, da die resultierenden quartären Ammoniumverbindungen schlecht wasserlöslich sind. Sie fallen bei der Herstellung in pastöser Form an und lassen sich aufgrund ihrer schlechten Wasserlöslichkeit nicht in industriellen Reinigern konfektionieren. Außerdem zeigte sich, daß Ammonium-Kationen, die zahlreiche Hydroxyalkyl-Gruppen enthalten, in nicht vollentsalztem Wasser störende Ausfällungen verursachen, was den Einsatz von derartigen Kation-Tensiden ebenfalls unmöglich macht. Weiterhin wird von kationischen Tensiden häufig eine demulgierende und/ oder entschäumende Wirkung auf Emulsionen und/oder anionische Tenside bzw. Emulgatoren erwartet. Die in der genannten Anmeldung offenbarten quartären Ammoniumverbindungen hatten jedoch keine demulgierende Wirkung auf Emulsionen und/oder anionische Emulgatoren. Diese Nachteile wurden auch nicht durch deutlich verbesserte Korrosionsschutz-Eigenschaften der hergestellten Kationtenside kompensiert; verglichen mit handelsüblichen kationischen Tensiden ergab sich ein nur annähernd gleich guter Korrosionsschutz.

In der EP-A-75 065 wird ein Verfahren zur Herstellung von quartären Ammoniumverbindungen beschrieben, bei welchem man ein tertiäres Amin mit einer Epoxyverbindung, die wenigstens eine endständige Epoxygruppe aufweist, in der Hitze bei Normaldruck umsetzt. Hierbei wird das tertiäre Amin zunächst zum Teil als Salz und zum Teil als freies Amin eingesetzt; die zur vollständigen Salzbildung benötigte Säuremenge wird am Ende der Umsetzung zugefügt. Als Säurekomponente kommen neben anorganischen Säuren auch organische Säuren in Frage. Die so gewonnenen quartären Ammoniumverbindungen finden Verwendung als Textilweichmacher oder - antistatika beziehungsweise als Antimikrobika, beispielsweise in flüssigen Mitteln zur Wäschenachbehandlung.

Aufgabe der vorliegenden Erfindung war es demgegenüber, neue, verbesserte Kationtenside auf der Basis von quartären Ammoniumverbindungen zur Verfügung zu stellen, die die Nachteile aus dem Stand der Technik nicht aufweisen. Insbesondere sollten Kationtenside für industriell verwendbare Reinigungsmittel zur Verfügung gestellt werden, deren Bestandteile den Korrosionsprozeß inhibieren, für eine ausreichende demulgierende Wirkung bezüglich anionischer Kontamination Sorge tragen und in wäßrigen industriellen Reinigern gut konfektioniert werden können, d.h. leicht wasserlöslich sind, keine störenden Ausfällungen verursachen und mit anderen üblicherweise in Industriereinigern verwendeten Komponenten verträglich

sind.

Die Aufgabe wird gelöst durch neue, verbesserte Kationtenside auf der Basis von quartären Ammoniumverbindungen, deren Ammonium-Stickstoffatom mindestens zwei Alkylreste, einen aus der Umsetzung mit einem 10 bis 24 C-Atome tragenden endständigen Epoxid stammenden 2-Hydroxyalkylrest und gegebenenfalls eine Arylalkylgruppe trägt und dessen Anion das Anion einer organischen Carbonsäure ist, das aus der Gruppe Benzoesäure, monosubstituierte Benzoesäuren, aliphatische Dicarbonsäuren, Fumarsäure, Maleinsäure und Sulfobernsteinsäure ausgewählt ist. Es wurde nämlich überraschend gefunden, daß derartige Kationtenside nicht nur gute demulgierende Eigenschaften bezüglich anionischer Tenside bzw. Emulgatoren aufweisen, sondern auch in entsprechenden Industriereinigern die behandelten Oberflächen hydrophobieren, zur Vermeidung von Korrosion dadurch beitragen, daß die Anwendungsflüssigkeiten gut und läuferfrei von den behandelten Flächen ablaufen und auf Kunststoffoberflächen sogar ein Antistatik-Effekt erzielt werden kann.

Gegenstand der Erfindung sind somit neue, verbesserte Kationtenside auf der Basis von quartären Ammoniumverbindungen, die dadurch gekennzeichnet sind, daß sie die allgemeine Formel

$$R^1\text{-CHOH-CHR}^2\text{-}\overset{+}{N}R^3R^4R^5 \quad X^- \qquad (I)$$

aufweisen, in der

- $R^1$ ein linearer oder verzweigter Alkylrest mit 1 bis 22 C-Atomen,
- $R^2$ Wasserstoff oder ein linearer oder verzweigter Alkylrest mit 1 bis 21 C-Atomen sein kann, wobei die Gesamtzahl der C-Atome der Substitutenten $R^1$ und $R^2$ im Bereich von 8 bis 22 liegt, und
- $R^3$ und $R^4$ für Methyl, Ethyl, 2-Hydroxyethyl oder 2-Hydroxypropyl,
- $R^5$ für Alkylreste mit 4 bis 6 C-Atomen oder Phenalkylreste mit 1 bis 3 C-Atomen im Alkylrest

und

- $X^-$ für das Anion der Benzoesäure, der mit $CH_3$, $NH_2$, $NO_2$, COOH, OH oder $SO_3H$ monosubstituierten Benzoesäure, einer aliphatischen Dicarbonsäure der allgemeinen Formel $HOOC\text{-}(CH_2)_n\text{-}COOH$, worin n = 2 bis 8 ist, der Fumarsäure, der Maleinsaure oder der Sulfobernsteinsäure

stehen.

Beispiele für lineare oder verzweigte Alkylreste, für die $R^1$ und $R^2$ stehen können, sind Methyl, Ethyl, Propyl, Isopropyl, n-Butyl, tert.-Butyl, Pentyl, Hexyl, Heptyl, Octyl, Nonyl, Decyl, Dodecyl, Tetradecyl und Hexadecyl. Bevorzugt werden quartäre Ammoniumverbindungen, in denen $R^2$ für Wasserstoff und $R^1$ für einen linearen oder verzweigten Alkylrest mit 8 bis 22 C-Atomen, z.B. n-Octyl, n-Decyl, n-Dodecyl, n-Tetradecyl oder n-Hexadeyl stehen. In jedem Fall muß die Gesamtzahl der C-Atome beider Substituenten $R^1$ und $R^2$ im Bereich von 8 bis 22 C-Atomen liegen. Hierbei ist es besonders bevorzugt, daß der Rest $R^1$ ein Decyl- oder Tetradecylrest ist.

Im Sinne der Erfindung ist es weiterhin bevorzugt, daß die Reste $R^3$ und $R^4$ in der allgemeinen Formel I für Methylgruppen stehen.

Der am Ammonium-Stickstoffatom gebundene Rest $R^5$ steht für Alkylreste wie beispielsweise n-Butyl, i-Butyl, tert.-Butyl, n-Pentyl, tert.-Butylmethyl oder n-Hexyl oder für Phenalkylreste wie Benzyl, Phenylethyl oder Phenylpropyl. Bevorzugt steht der Rest $R^5$ in der allgemeinen Formel I für einen Benzyl- oder n-Butylrest.

Das Gegenanion in den erfindungsgemäßen Kationtensiden ist das Anion einer organischen Carbonsäure, d.h. der Rest $X^-$ in der allgemeinen Formel I steht für das Anion der Benzoesäure, der mit $CH_3$, $NH_2$, $NO_2$, COOH oder $SO_3H$ monosubstituierten Benzoesäure, einer aliphatischen Dicarbonsäure der allgemeinen Formel $HOOC\text{-}(CH_2)_n\text{-}COOH$, worin n=2 bis 8 ist, d.h. der Bernsteinsäure, der Glutarsäure, der Adipinsäure, der Pimelinsäure, der Korksäure, der Azelainsäure, der Sebacinsäure, der Fumarsäure, der Maleinsäure und der Sulfobernsteinsäure. Besonders bevorzugt werden die Anionen der Benzoesäure und der Fumarsäure.

Je nach der Wertigkeit des Anions, d. h. der Basizität der entsprechenden organischen Carbonsäure, kann das Kation der allgemeinen Formel (I) in den erfindungsgemäßen quartären Ammoniumverbindungen auch mehrfach enthalten sein. Somit werden von der allgemeinen Formel (I) sowohl die entsprechenden neutralen als auch die entsprechenden sauren Salze umfaßt.

Vorzugsweise werden in industriellen Reinigungsmitteln folgende Einzelverbindungen verwendet: Benzyldimethyl-2-hydroxydodecylammoniumbenzoat und Bis-(benzyldimethyl-2-hydroxydodecylammonium)fumarat. Von diesen wird Benzyldimethyl-2-hydroxydodecylammoniumbenzoat besonders bevorzugt.

Die erfindungsgemäßen Verbindungen der allgemeinen Formel I lassen sich nach an sich bekannten Methoden dadurch herstellen, daß man das Salz eines tertiären Amins der allgemeinen Formel

$NR^3R^4R^5$ (II)

in der $R^3$, $R^4$ und $R^5$ die oben angegebenen Bedeutungen haben, und einer organischen Säure der allgemeinen Formel

$X^-H^+$ (III)

in der $X^-$ die oben angegebene Bedeutung hat, in Wasser mit einer Epoxidverbindung der allgemeinen Formel

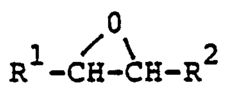
(IV)

in der $R^1$ und $R^2$ die oben angegebenen Bedeutungen haben und zusammen eine Anzahl von C-Atomen im Bereich von 8 bis 22 aufweisen, in stöchiometrischem Verhältnis bei Normaldruck und einer Temperatur zwischen 40 und 100°C umsetzt, wobei das Reaktionsgemisch vor Beginn der Umsetzung einen pH-Wert von wenigstens 7 aufweist.

Die zur Herstellung der erfindungsgemäßen quartären Ammoniumverbindungen verwendeten Epoxide der allgemeinen Formel IV können also Epoxide mit 10 bis 24 C-Atomen sein, in denen sich der Oxiranring an jeder beliebigen Stelle des Moleküls befindet. Bevorzugt werden allerdings quartäre Ammoniumverbindungen, die durch Umsetzung des Amin-Salzes mit einem 1.2-Epoxid hergestellt wurden, d.h. Verbindungen der allgemeinen Formel I, in der $R^1$ ein Alkylrest mit 8 bis 22 C-Atomen und $R^2$ ein Wasserstoffatom ist.

Die zur Herstellung der quartären Ammoniumverbindungen der allgemeinen Formel I verwendeten Amine sind vorzugsweise tertiäre Alkyl-, Hydroxyalkyl- oder Alkylarylamine, wobei Dimethylbutylamin und Dimethylbenzylamin besonders bevorzugt werden.

Die erfindungsgemäßen quartären Ammoniumverbindungen werden als Kationtenside in industriellen Reinigerlösungen verwendet. Sie haben dabei gegenüber anderen Kationtensiden wie auch schon aus dem Stand der Technik bekannten quartären Ammoniumverbindungen den Vorteil, daß sie keine korrosiven oder zu unerwünschten Ausfällungen führenden Gegenanionen enthalten. Die Anionen der für die Herstellung der erfindungsgemäßen Ammoniumverbindungen verwendeten organischen Säure sind im Gegensatz dazu sogar in der Lage, den Korrosionsprozeß auf gereinigten Metalloberflächen zu inhibieren. Durch die fehlende Anreicherung korrosiver Anionen in den Bädern und die inhibierende Wirkung der Carbonsäure-Anionen wird also eine Verbesserung der Korrosionsschutzeigenschaften in wäßrigen Medien erzielt. Dies gilt gleichermaßen für Teile, die vor dem Weiterverarbeitungsprozeß zwischengelagert werden müssen, als auch für Teile, die unmittelbar weiterverarbeitet werden. Der verbesserte Korrosionsschutz macht sich insbesondere bei solchen - mit quartäre Ammoniumverbindungen enthaltenden Industriereinigern behandelten - Teilen bemerkbar, die aufgrund ihrer Geometrie schöpfend wirken. Bei Verwendung herkömmlicher Reiniger war die Korrosionsgefahr auf solchen Teilen besonders groß, da bei Verdunsten des Lösungsmittels, in der Regel Wasser, eine starke Anreicherung der Inhaltsstoffe und damit auch der korrosiven Bestandteile zu befürchten war. Dies wird bei Verwendung von die erfindungsgemäßen quartären Ammoniumverbindungen als Kationtenside enthaltenden industriellen Reinigerlösungen verhindert.

Da die erfindungsgemäßen Kationtenside gut wasserlöslich sind, sind sie auch gut konfektionierbar und rahmen in der Anwendungslösung nicht auf. Entsprechende Anwendungslösungen zeigen auch ein günstiges Ablaufverhalten.

Einen weiteren Vorteil bieten die erfindungsgemäßen Kationtenside insofern, als eine vorteilhafte Hydrophobierung der gereinigten Oberflächen, insbesondere der gereinigten Metalloberflächen, beobachtet wird. Weiterhin wird durch ein sehr gutes Ablaufverhalten der Anwendungsflüssigkeit Korrosion der behandelten Teile vermieden.

Es wurde weiterhin beobachtet, daß sich wäßrige Industriereiniger-Lösungen, die die erfindungsgemäßen quartären Ammoniumverbindungen als Kationtenside enthalten, auch zur Kunststoffreinigung einsetzen lassen, da sie eine antistatische Wirkung aufweisen. Gerade dies erschließt derartigen Produkten in der Zukunft einen weiten Anwendungsbereich, da auch Kunststoffflächen analog Metallflächen mit steigender

Tendenz in Spritzverfahren gereinigt werden.

Die erfindungsgemäßen quartären Ammoniumverbindungen eignen sich zur Verwendung in allen für die industrielle Reinigung wichtigen Reinigungsmitteln. So können sie in spritzfähigen Reinigern, z.B. neutralen bis schwach alkalischen Reinigern oder sauren Reinigern enthalten sein, insbesondere in solchen Reinigungslösungen, die unter hohem Druck auf die zu reinigenden Teile aufgespritzt werden. Gleichermaßen sind sie jedoch mit Vorteil auch in Tauchreinigern auf der Basis nichtionischer Tenside zu verwenden.

Quartäre Ammoniumverbindungen gemäß der Erfindung werden ebenfalls als kationische Tenside eingesetzt, die in Industriereinigerlösungen zum Spritzen oder Tauchen als Demulgatoren oder Antischaummittel wirken.

Die neuen verbesserten Kationtenside auf der Basis von quartären Ammoniumverbindungen werden nach an sich bekannten Verfahren mit - für industrielle Reinigungslösungen üblichen - weiteren Bestandteilen konfektioniert. Diese Lösungen können neben den quartären Ammoniumverbindungen und üblichen Inhaltsstoffen gegebenenfalls noch weitere Zusätze, z.B. an Alkanolaminen, Phosphaten, Boraten oder Nitriten enthalten. Erwünschtenfalls können den Lösungen auch Inhibitoren, insbesondere für Nichteisenmetalle, oder Biozide wie beispielsweise Hexahydrotriazinderivate und/oder Phenole und/oder Chlorphenole zur Verhinderung von Bakterien-und/oder Pilzbefall in den Spritz- oder Tauchanlagen zugesetzt werden.

Die Erfindung wird durch die nachstehenden Beispiele weiter erläutert.

Hierin bedeuten die verwendeten Abkürzungen "EO" = Ethylenoxid und "PO" = Propylenoxid. Die vor diesen Abkürzungen stehenden Zahlen geben die Anzahl der Mole des jeweiligen Alkylenoxids wieder, welche in dem betreffenden Anlagerungsprodukt enthalten sind.

Beispiel 1
(Herstellung von Benzyl-2-hydroxydodecyl-dimethyl-ammonium-benzoat)

In einen Dreihalskolben mit Rührer, Kontaktthermometer und Rückflußkühler wurden
106,7 g Wasser
135,2 g (1,0 mol) Dimethylbenzylamin
97,7 g (0,8 mol) Benzoesäure und
188,0 g (1,0 mol) 1,2-Epoxydodecan(EpOZ 8,551, daraus ber. M 188,0)
in der angegebenen Reihenfolge unter Rühren zusammengegeben und anschließend auf 95 °C aufgeheizt.

Nach wenigen Minuten war eine klare Lösung entstanden, die nach 15 Minuten mit der Restmenge
24,4 g (0,2 mol) Benzoesäure
versetzt und 6 Std. bei 95 °C gerührt wurde. Die Lösung war ca. 80%ig und besaß folgende Kennzahlen:

| SZ | EpOZ | AZ | Epton-Wert | Barr-Wert |
|---|---|---|---|---|
| 3,6 | 0,002 | 104,1 | 132,2 mval/100g | 128 mval/100g |

Beispiel 2
(Herstellung von Bis-(benzyl-2-hydroxydodecyl-dimethyl-ammonium)-fumarat)

In einer Rührapparatur, bestehend aus Dreihalskolben, Kontaktthermometer und Rückflußkühler wurden
81,25 g (0,7 mol) Fumarsäure in
126,98 g Wasser gelöst und
184,30 g (1,4 mol) Dimethylbenzylamin, ferner
263,20 g( 1,4 mol) 1,2-Epoxydodecan hinzugefügt.

Das Gemisch wurde unter ständigem Rühren auf 95 °C erwärmt und 4,5 Std. auf dieser Temperatur gehalten.

Die entstandene gelbliche, ca. 80 %ige Lösung war nach dem Auskühlen zähflüssig und besaß folgende Kennzahlen:

| SZ | EpOZ | AZ | Epton-Wert | Barr-Wert |
|---|---|---|---|---|
| 14,4 | 0,07 | 112 | 138 | 112 mval/100g |

Beispiel 3
(Herstellung von Benzyldimethyl-2-hydroxydodecylammoniumsuccinat)

In die in Beispiel 2 beschriebene Apparatur wurden der Reihe nach 109,4 g Wasser, 135,2 g (1,0 mol) Dimethylbenzylamin, 118,1 g (1,0 mol) Bernsteinsäure (Säurezahl 950,2) und 190,9 g (1,0 mol) 1,2-Epoxydodecan (EpOZ 8,38, daraus ber. M 190,9) gegeben. Nach 4,5-stündigem Rühren bei 95° C war eine einphasige Lösung entstanden. Beim Abkühlen wurde das Produkt trübe; Epoxidzahl: 0,10; Säurezahl: 118,6.

Beispiel 4
(Herstellung von n-Butyldimethyl-2-hydroxydodecylammoniummaleat)

In der gleichen Apparatur und unter gleichen Bedingungen wie in Beispiel 2 wurden folgende Reaktionsteilnehmer umgesetzt: 93,8 g Wasser, 101,2 g (1,0 mol) Dimethylbutylamin, 116,1 g (1,0 mol) Maleinsäure (Säurezahl 966,8) und 190,9 g (1,0 mol) 1,2-Epoxydodecan (EpOZ 8,38). Es entstand eine gelbe, in der Hitze klare und nach Abkühlen trübe 80 %ige Lösung des Produktes (Epoxidzahl: 0,01; Säurezahl: 118,8; Epton-Wert: 125,1 mval/100 g).

Beispiel 5
(Herstellung von Benzyldimethyl-2-hydroxyhexadecylammonium-sulfosuccinat)

In der gleichen Apparatur und unter gleichen Bedingungen wie in Beispiel 2 wurden folgende Reaktionsteilnehmer umgesetzt: 68,1 g Wasser, 67,6 g (0,5 mol) Dimethylbenzylamin, 99.1 g (0,5 mol) Sulfobernsteinsäure (Säurezahl 849,5) und 123,8 g (0,5 mol), 1,2-Epoxyhexadecan (Epoxidzahl 6,46). Nach Abkühlen auf Raumtemperatur erstarrte die in der Hitze klare, gelbe Lösung zu einer halbfesten Masse; diese enthielt das Produkt in einer Konzentration von 81 % (Epoxidzahl: 0,06; Säurezahl: 161,3; Epton-Wert: 132,7 mval/100 g).

Beispiel 6

Es wurden Reinigerlösungen mit Anwendungskonzentrationen im Bereich von 0,5 bis 5 % hergestellt, die für im Spritzen aufzubringende Reiniger vorgesehen waren. Die Lösungen hatten die folgenden Zusammensetzungen (Angaben in Gew.-%):

a) Neutralreiniger

1. 30 % Triethanolamin,
10% Caprylsäure,
5 % Hexahydrotriazinderivat,
0,5% Tolyltriazol,
4% eines Anlagerungsproduktes von 2 EO und
4 PO an einen Alkohol mit 18 C-Atomen,
1 % eines Anlagerungsproduktes von 5 EO und
30 PO an 1.2-Propylenglykol,
1,5% Benzyldimethyl-2-hydroxydodecylammoniumbenzoat und
48,0% vollentsalztes Wasser.

2. 10 % Natriumcaprylat,
10% Triethanolamin,
5 % Borax,
10 % Natriumtriphosphat,
4 % eines Anlagerungsproduktes von 9 EO und
10 PO an Nonylphenol,
2 % Bis-(benzyldimethyl-2-hydroxydodecylammonium)fumarat und
59,0% vollentsalztes Wasser.

b) Alkalischer Industriereiniger

15% Kaliumtriphosphat,
6 % Triethanolamin,
5 % Kaliumhydroxid,
2 % eines Anlagerungsproduktes von 3 EO und
6 PO an einen Alkohol mit 12 bis 18 C-Atomen,

4 % Isononansäure,
2 % Bis-(benzyldimethyl-2-hydroxydodecylammonium)succinat und
66 % vollentsalztes Wasser.

c) Saure Reiniger

1. 25 % Natriumdihydrogenphosphat,
1 % Benzoesäure,
0,2% Natriumolybdat,
1 % Butyldimethyl-2-hydroxydodecylammoniumbenzoat,
3 % nichtionogenes Tensid Triton DF16® und
69,8% vollentsalztes Wasser.

2. 12 % Diethanolamin,
15% 2-Phosphonobutan-1.2.4-tricarbonsäure,
5 % Gluconsäure,
3 % nichtionogenes Tensid Triton DF16®,
8 % Fettalkoholsulfat in Form des Natriumsalzes,
3 % Phosphorsäure,
2 % Bis-(benzyldimethyl-2-hydroxydodecylammonium)fumarat und
52 % vollentsalztes Wasser.

Die wie oben beschrieben formulierten Reinigungsmittel konnten gut im Spritzen aufgetragen werden. Sie zeigten geringe bzw. gar keine Neigung zum Schäumen. Die Reinigungsbäder waren über längere Zeit stabil und ließen über diese Zeit in ihrer Reinigungskraft nicht nach. Aufgrund der Verwendung der erfindungsgemäßen quartären Ammoniumverbindungen korrodierten die mit den Reinigern im Spritzen behandelten Metalloberflächen nicht, sondern wiesen im Vergleich zu einer Behandlung mit herkömmlichen Reinigern erhöhten Korrosionssohutz auf.

Beispiel 7

Alkalische, industrielle Tauchreiniger; Anwendungskonzentrationen im Bereich von 1 bis 7 %.

1. 15 % Kaliumtriphosphat,
6 % Triethanolamin,
6 % Isononansäure,
1,5% Benzyldimethyl-2-hexadecylammoniumbenzoat,
10 % Diethanolamin,
5 % eines Anlagerungsproduktes von 10 EO an Nonylphenol und
56,5% vollentsalztes Wasser.

2. 40 % Natriumdiphosphat,
30 % Natriumorthophosphat,
10 % Natriumtriphosphat,
10% Natriummetasilicat,
1 % Bis-(benzyldimethyl-2-hydroxydodecylammonium)fumarat und
9 % eines Anlagerungsproduktes von 10 EO an Nonylphenol.

Die wie oben beschrieben formulierten Reinigungsmittel hatten eine über längere Zeit hohe Reinigungskraft auf behandelten Metalloberflächen, und ihre Bäder wiesen eine hohe Stabilität auf. Metalloberflächen, die mit den oben beschriebenen Reinigern im Tauchen behandelt worden waren, wiesen einen verbesserten Korrosionsschutz im Vergleich zu mit herkömmlichen Reinigern behandelten Metalloberflächen auf.

Beispiel 8
Demulgierende Wirkung

A. Neutralreiniger

Beschrieben wird die demulgierende Wirkung eines Neutralreinigers auf folgender Basis:

35% Alkanolamin - $C_8$-$C_{12}$-Carboxylat
2 % 1-Hydroxyethan-1,1-diphosphonsäure
0,5 % Mercaptobenzthiazol
3 % Blockpolymerisat aus Ethylendiamin + 30 Mol Ethylenoxid sowie 60 Mol Propylenoxid
1 % Anlagerungsprodukt von 10 Mol Ethylenoxid an $C_{12}$-$C_{16}$-Fettamin
58,5 % Wasser

a. Emulsionsspaltung

Die Emulsionsspaltung wurde nach folgendem Test durchgeführt:
10 g Bohröl wurden bei Raumtemperatur mit 40 g einer 2%igen wäßrigen Neutralreinigerlösung in einem 270 ml Ölabscheidungskolben emulgiert. Eine äquivalente Menge BDHA-benzoat *) wurde zugegeben. Unter Schütteln wurde mit 2%iger heißer Neutralreinigerlösung aufgefüllt. Die Demulgation setzte spontan ein. Zur vollständigen Ölseparierung aus der sich zunächst abscheidenden "emulsionsartigen Phase" temperte man die Flüssigkeit zwei Stunden bei 80° C.

Ergebnis:

Das Öl wurde praktisch quantitativ abgetrennt.

b. Zusatz von Antischaummittel:

10 g Bohröl wurden bei Raumtemperatur mit 40 g einer 2%igen wäßrigen Neutralreinigerlösung in einem 270 ml Ölabscheidungskolben emulgiert.
BDHA-benzoat wurde dieser Emulsion in stöchiometrischem Überschuß (Verhältnis Demulgator zu anionischem Emulgator = 1,2 : 1) zugegeben. Ferner wurde das Anlagerungsprodukt von 30 Mol Ethylenoxid und 60 Mol Propylenoxid an Ethylendiamin als Antischaummittel zugegeben (Verhältnis Demulgator : Antischaummittel = 1 : 1). Unter Schütteln wurde mit 2%iger wäßriger Neutralreinigerlösung aufgefüllt. Die Spaltung setzte spontan ein. Die rückgeführte Reinigungslösung konnte schaumfrei gespritzt werden.

B. Alkalische Reinigungsmittel

Beschrieben wird die Spaltung von Emulsionen in Gegenwart alkalischer Reinigungsmittel, die die jeweils einleitend angegebene Zusammensetzung hatten:

a) 63 % Natriummetasilikat
14 % Natriumhydroxid
15 % Soda
2 % Fettalkohol + 14 Ethylenoxid
3 % Alkylbenzolsulfonat
Zu einer 4%igen Reinigerlösung in Leitungswasser wurden bei Raumtemperatur 2 % Bohröl-Konzentrat emulgiert, eine mehr als äquivalente Menge (1 : 1,1) BDHA-benzoat zugegeben und ca. 3 min. gut gerührt. Man ließ die Mischung stehen, die Separierung des Öls begann sofort.
b) 60 % Natriummetasilikat
10 % Natriumhydroxid
15% Soda
10% Natriumdiphosphat
2,5 % Fettalkohol + 14 Ethylenoxid
2,5 % Kokosamin + 12 Ethylenoxid
Wie unter a) beschrieben, wurde zu einer 3%igen Reinigerlösung eine doppelt äquivalente Menge BDHA-benzoat zur Emulsionsspaltung gegeben. Die Separierung begann sofort, wobei die wäßrige Phase nahezu klar war.
c) 50 % Natriumdiphosphat
15 % Natriumtriphosphat
15 % Trinatriumphosphat
10 % Soda
7,7 % Nonylphenol + 14 Ethylenoxid
2,3 % Kokosfettsäuremonoethanolamid + 4 Ethylenoxid
Wie unter a) und b) beschrieben, wurde einer belasteten 2%igen Reinigerlösung eine doppelt äquivalente Menge BDHA-benzoat zugegeben. Die Separierung erfolgte spontan, wobei die wäßrige Phase nahezu klar wurde.
Die Anwesenheit von Pyro- oder Polymerphosphaten und/oder anionischen Tensiden bedingte eine höhere Zugabe an Demulgator zur vollständigen und raschen Demulgierung.

*) BDHA = Benzyldimethyl-2-hydroxydodecyl-ammonium-Ion.

Beispiel 9

Allgemeine Reinigungsmittel (z.B. Reiniger für Fahrzeuge, Reiniger für Wände und Fußböden von Industriebetrieben und Produkte für die Dampfstrahlreinigung; Anwendungskonzentrationen im Bereich von 2 bis 30 %)

1. 8 % eines Anlagerungsproduktes von 14 EO an einen Alkohol mit 12 bis 14 C-Atomen,
7 % Fettalkoholsulfat,
3 % Butyldimethyl-2-hydroxydodecylammoniumbenzoat,
5 % Kaliumhydroxid,
10 % Diethanolamin,
6 % Phosphorsäure und
61 % vollentsalztes Wasser.
2. 8 % Natriumtriphosphat,
5 % Isononansäure,
5 % Borsäure,
8 % Monoethanolamin,
1 % Kaliumhydroxid,
5 % eines Anlagerungsproduktes von 12 EO an ein Amin mit 12 C-Atomen,
3 % Bis-(benzyldimethyl-2-hydroxydodecylammonium)fumarat und
65 % vollentsalztes Wasser.

Die wie oben beschrieben zusammengesetzten Reinigungsmittel zeigten gute Reinigungswirkung und ein gleichmäßiges, läuferfreies Ablaufverhalten auf den behandelten Teilen.

Aufgrund der Verwendung der erfindungsgemäßen quartären Ammoniumverbindungen war der Korrosionsschutz für mit den Reinigungslösungen behandelte Metallflächen und -gegenstände im Vergleich zu der Behandlung mit herkömmlichen Reinigungsmitteln deutlich verbessert.

Beispiel 10
Vergleichender Korrosionstest

Hinsichtlich ihrer korrosionsinhibierenden Eigenschaften wurden Lösungen vergleichend getestet, die als quartäre Ammoniumverbindung das aus dem Stand der Technik bekannte Produkt Dehyquart® LDB (Fa. Henkel) und BDHA-benzoat gemäß der vorliegenden Erfindung enthielten.

Die Prüfung erfolgt gemäß dem Spänetest DIN 51360/2 mit 1 bis 3%igen Lösungen in vollentsalztem und 20° d-Wasser von Konzentraten der Rezepturen I und II:

I: 12,5 % Dehyquart® LDB (35 % Aktivgehalt)
37,5 % Diethanolamin
Rest Wasser
II: 5,5 % BDHA-benzoat (80 % Aktivgehalt)
37,5 % Diethanolamin
Rest Wasser

Die Ergebnisse sind der nachfolgenden Tabelle zu entnehmen.

| | Korrosionsgrad gemäß DIN 51360/2 bei Verwendung von | | | |
|---|---|---|---|---|
| | I:Dehyquart$^R$ LDB in | | 11: BDHA-benzoat in | |
| Konzentration der Lösung | V.E. Wasser | 20° d-Wasser | V.E. Wasser | 20° d-Wasser |
| 1 % | 2 | 4 | 0,5 | 3 |
| 2 % | 0 | 3 | 0 | 2 |
| 3 % | 0 | 2 | 0 | 0,5 |

Wie Spalten 4 und 5 zu entnehmen ist, zeigt BDHA-benzoat ein deutlich besseres Korrosionsschutzverhalten. (Dehyquart$^R$ LDB = Lauryldimethylbenzylammoniumchlorid)

**Patentansprüche**

1. Neue, verbesserte Kationtenside auf der Basis von quartären Ammoniumverbindungen, dadurch gekennzeichnet, daß sie die allgemeine Formel

$$R^1\text{-CHOH-CHR}^2\text{-}\overset{+}{N}R^3R^4R^5 \quad X^- \qquad (I)$$

aufweisen, in der

| | |
|---|---|
| $R^1$ | ein linearer oder verzweigter Alkylrest mit 1 bis 22 C-Atomen, |
| $R^2$ | Wasserstoff oder ein linearer oder verzweigter Alkylrest mit 1 bis 21 C-Atomen sein kann, wobei die Gesamtzahl der C-Atome der Substituenten $R^1$ und $R^2$ im Bereich von 8 bis 22 liegt, und |
| $R^3$ und $R^4$ | für Methyl, Ethyl, 2-Hydroxyethyl oder 2-Hydroxypropyl, |
| $R^5$ | für Alkylreste mit 4 bis 6 C-Atomen oder Phenalkylreste mit 1 bis 3 C-Atomen im Alkylrest, |

und

$X^-$ für das Anion der Benzoesäure, der mit $CH_3$, $NH_2$, $NO_2$, COOH, OH oder $SO_3H$ monosubstituierten Benzoesäure, einer aliphatischen Dicarbonsäure der allgemeinen Formel HOOC-$(CH_2)_n$-COOH, worin n = 2 bis 8 ist, der Fumarsäure, der Maleinsäure oder der Sulfobernsteinsäure

stehen.

2. Kationtenside nach Anspruch 1, dadurch gekennzeichnet, daß in der allgemeinen Formel I $R^1$ ein Alkylrest mit 8 bis 22 C-Atomen und $R^2$ ein Wasserstoffatom ist.

3. Kationtenside nach Anspruch 2, dadurch gekennzeichnet, daß in der allgemeinen Formel I $R^1$ ein Decyl-oder Tetradecylrest ist.

4. Kationtenside nach Anspruch 1, dadurch gekennzeichnet, daß $R^3$ und $R^4$ für Methylgruppen stehen.

5. Kationtenside nach Anspruch 1, dadurch gekennzeichnet, daß in der allgemeinen Formel I $R^5$ für einen Benzyl- oder n-Butylrest steht.

6. Kationtenside nach Anspruch 1, dadurch gekennzeichnet, daß in der allgemeinen Formel I $X^-$ für das Benzoesäureanion steht.

7. Kationtenside nach Anspruch 1, dadurch gekennzeichnet, daß in der allgemeinen Formel I $X^-$ für das Fumarsäureanion steht.

8. Benzyldimethyl-2-hydroxydodecylammoniumbenzoat.

9. Bis-(benzyldimethyl-2-hydroxydodecyl-ammonium)fumarat.

10. Verwendung der Kationtenside auf Basis von quartären Ammoniumverbindungen nach Ansprüchen 1 bis 9 in spritzfähigen Reinigern, Tauchreinigern, demulgierenden Zusätzen zu Industriereinigerlösungen oder sonstigen Reinigungsmitteln.

## Claims

1. New improved cationic surfactants based on quaternary ammonium compounds, characterized in that they correspond to the following general formula

$$R^1\text{-CHOH-CHR}^2\text{-}\overset{+}{N}R^3R^4R^5 \qquad X^- \qquad (I)$$

in which

| | |
|---|---|
| $R^1$ | is a linear or branched $C_{1-22}$ alkyl radical, |
| $R^2$ | is hydrogen or a linear or branched $C_{1-21}$ alkyl radical; the total number of carbon atoms in the substituents $R^1$ and $R^2$ is in the range from 8 to 22, and |
| $R^3$ and $R^4$ | represent methyl, ethyl, 2-hydroxyethyl or 2-hydroxypropyl, |
| $R^5$ | represents $C_{4-6}$ alkyl radicals or phenalkyl radicals containing 1 to 3 carbon atoms in the alkyl radical and |
| $X^-$ | is the anion of benzoic acid, benzoic acid monosubstituted by $CH_3$, $NH_2$, $NO_2$, COOH, OH or $SO_3H$, an aliphatic dicarboxylic acid corresponding to the general formula $HOOC-(CH_2)_n-COOH$, in which n = 2 to 8, fumaric acid, maleic acid or sulfosuccinic acid. |

2. Cationic surfactants as claimed in claim 1, characterzed in that, in general formula I, $R^1$ is a $C_{8-22}$ alkyl radical and $R^2$ is a hydrogen atom.

3. Cationic surfactants as claimed in claim 2, characterzed in that, in general formula I $R^1$ is a decyl or tetradecyl radical.

4. Cationic surfactants as claimed in claim 1, characterized in that $R^3$ and $R^4$ represent methyl groups.

5. Cationic surfactants as claimed in claim 1, characterized in that, in general formula I, $R^5$ is a benzyl or n-butyl radical.

6. Cationic surfactants as claimed in claim 1, characterized in that, in general formula I, $X^-$ is the benzoic acid anion.

7. Cationic surfactants as claimed in claim 1, characterized in that, in general formula I, $X^-$ is the fumaric acid anion.

8. Benzyldimethyl-2-hydroxydodecyl ammonium benzoate.

9. Bis-(benzyldimethyl-2-hydroxydodecyl ammonium)-fumarate

10. The use of the cationic surfactants based on quaternary ammonium compounds claimed in claims 1 to 9 in sprayable cleaning solutions, dip cleaning solutions, demulsifying additives for industrial cleaning solutions or other cleaning preparations.

**Revendications**

1. Nouveaux agents tensioactifs cationiques améliorés à base de composés d 'ammonium quaternaire, caractérisés en ce qu'ils présentent la formule générale suivante :

$$R^1\text{-CHOH-CHR}^2\text{-}\overset{+}{N}R^3R^4R^5 \quad X^- \qquad (I)$$

dans laquelle

| | |
|---|---|
| $R^1$ | est un radical alkyle linéaire ou ramifié à 1 à 22 atomes de carbone, |
| $R^2$ | peut être de l'hydrogène ou un radical alkyle linéaire ou ramifié à 1 à 21 atomes de carbone, tandis que le nombre total des atomes de carbone des substituants $R^1$ et $R^2$ est compris dans la gamme de 8 à 22, et |
| $R^3$ et $R^4$ | représentent un radical méthyle, éthyle, 2-hydroxyéthyle ou 2-hydroxypropyle, |
| $R^5$ | représente des radicaux alkyle à 4 à 6 atomes de carbone ou des radicaux phénalkyle à 1 à 3 atomes de carbone dans le radical alkyle, |

et

$X^-$ est l'anion de l'acide benzoïque, de l'acide benzoïque monosubstitué par $CH_3$, $NH_2$, $NO_2$, COOH, OH ou $SO_3H$, d'un acide dicarboxylique aliphatique de la formule générale

HOOC-$(CH_2)_n$-COOH

dans laquelle n est compris entre 2 et 8, de l'acide fumarique, de l'acide maléique ou de l'acide sulfosuccinique.

2. Agent tensioactif cationique selon la revendication 1 caractérisé en ce que, dans la formule générale (I), $R^1$ représente un radical alkyle à 8 à 22 atomes de carbone et $R^2$ un atome d'hydrogène.

3. Agent tensioactif cationique selon la revendication 2 caractérisé en ce que, dans la formule générale (I), $R^2$ représente un radical décyle ou tétradécyle.

4. Agent tensioactif cationique selon la revendication 1 caractérisé en ce que $R^3$ et $R^4$ représentent des groupes méthyle.

5. Agent tensioactif cationique selon la revendication 1 caractérisé en ce que, dans la formule générale (I), $R^5$ représente un radical benzyle ou n-butyle.

6. Agent tensioactif cationique selon la revendication 1 caractérisé en ce que, dans la formule générale (I)-=, X représente l'anion de l'acide benzoïque.

7. Agent tensioactif cationique selon la revendication 1 caractérisé en ce que, dans la formule générale (I), X représente l'anion de l'acide fumarique.

8. Benzoate de benzyldiméthyl-2-hydroxydodécylammonium.

9. Bis-fumarate de (benzyldiméthyl-2-hydroxydodécylammonium).

10. Utilisation des agents tensioactifs cationiques à base de composés d'ammonium quaternaire selon les revendications 1 à 9, dans des produits de nettoyage pour pulvérisation, pour trempage,dans les additifs de désémulsification pour les solutions de nettoyage industriel ou pour d'autres produits de nettoyage.